Europäisches Patentamt

European Patent Office

Office européen des brevets

(19)

(11) Publication number: **0 128 197**

**B1**

(12) EUROPEAN PATENT SPECIFICATION

(45) Date of publication of patent specification: 08.06.88

(51) Int. Cl.⁴: **F 04 B 43/04**

(21) Application number: 84900226.6

(22) Date of filing: 30.11.83

(88) International application number:
PCT/US83/01891

(87) International publication number:
WO 84/02164 07.06.84 Gazette 84/14

(54) PUMP ACTUATOR.

(30) Priority: 03.12.82 US 446454
03.12.82 US 446455

(43) Date of publication of application:
19.12.84 Bulletin 84/51

(45) Publication of the grant of the patent:
08.06.88 Bulletin 88/23

(84) Designated Contracting States:
AT BE CH DE FR GB LI LU NL SE

(56) References cited:
EP-A-0 053 437
AU-B- 482 500
US-A-1 893 776
US-A-2 228 565
US-A-2 429 441
US-A-4 167 046
US-A-4 384 829

No relevant documents have been disclosed

(73) Proprietor: Novacor Medical Corporation
7799 Pardee Lane
Oakland, CA 94621 (US)

(72) Inventor: MILLER, Phillip, Jay
968 Regal Road
Berkeley, CA 94708 (US)
Inventor: JASSAWALLA, Jal, Sohrab
66 Cleary Court, No. 806
San Francisco, CA 94109 (US)
Inventor: CHEN, Herbert
7 Ardmore Path
Kensington, CA 94707 (US)
Inventor: CONLEY, Michael, George
118 Behrens Street
El Cerrito, CA 94530 (US)

(74) Representative: Bayliss, Geoffrey Cyril et al
BOULT, WADE & TENNANT 27 Furnival Street
London EC4A 1PQ (GB)

Courier Press, Leamington Spa, England.

## Description

The present invention relates to an actuator mechanism for a pump, and in particular, to an improved actuator mechanism and fluid chamber structure for use in an internally implantable blood pump or the like.

In any type of blood pump, the device must be constructed in size, shape and design to be readily implanted in a body cavity and to be comfortable over extended periods. The construction of the device must allow for efficient connections between the pump and the vascular system near the heart. The pump must be highly reliable for long periods of continuous use. Specifically, the actuator mechanism in the pump must be efficient, long-lasting, and relatively smooth working. The chambered part of the pump — which in the case of a deformable-sac pump, includes the deformable sac itself — must be constructed to operate over long periods of continuous use with minimum stress-related fatigue and creasing. The sac design and operating characteristics must minimize thrombus formation in the fluid-containing portions of the pump. Additionally, the sac itself should be easy and relatively inexpensive to manufacture and incorporate into a rigid housing portion of the pump.

US—A—4,167,046 discloses a deformable sac blood pump which solves or minimizes a number of problems associated with previously developed deformable sac· blood pumps. The pump of that invention is composed of a disc-like deformable sac having an annular side wall and a pair of opposed circular walls which are adapted to be recurrently and sychronously moved toward one another, through the action of a pair of opposed pusher plates to produce pumping action. Inlet and outlet conduits in the sac are arranged to direct inlet and outlet flow substantially tangentially with respect to the annulus formed by the inner surface of the sac's annular side wall. The inlet and outlet conduits are disposed on either side of the pump region occupied by the pump actuator, producing a space-conserving arrangement of pump components. The sac itself is formed as a unitary seamless article from flexible resilient material, providing a smooth interior sac surface which acts to prevent thrombus formation in the pump.

The actuator which is used in a deformable-sac pump of the type just described operates to move the opposed pusher plates recurrently from their more displaced to their less displaced positions, preferably in a manner which produces controlled-rate, synchronous plate movement. At the same time, the actuator must be efficient, relatively smooth working, and reliable over long periods of continued use. One type of actuator which provides a number of advantages in a deformable-blood sac has been described in US—A—4,384,829 (& EP—A—0 053 437).

The actuator disclosed in the just-mentioned patent includes a solenoid device which operates between open and closed positions to produce increased bending in opposed prestressed beam springs which operatively connect opposed armatures in the actuator to associated pusher plates. The increased bending in the springs is released by movement of the two pusher plates inwardly toward their less displaced end-of-stroke positions. An important feature of the actuator is that the beam springs, in acting between less stressed and more stressed conditions, produce a relatively flat output force profile in acting against the associated pusher plates. As a result, peak loads on the pump components are minimized. The actuator, having few moving parts, is also simple in construction and operation, and is therefore quite reliable over. long periods of continued use.

The present invention contemplates an improved actuator and sac which incorporate many of the advantageous features of the actuator and sac just described, and further include a number of unique and hitherto unknown features which enhance pump reliability and operational characteristics and contribute to compactness in the pump design.

Accordingly, one object of the present invention is to provide an actuator for use in a deformable-sac type implantable blood pump, where the actuator is quite compact.

Another object of the invention is to provide such an actuator having highly reliable and predictable performance characteristics.

It is yet another object of the invention to provide such an actuator whose operation can be accurately monitored and controlled.

Another object of the present invention is to provide in a deformable sac blood pump, a chamber structure which incorporates many of the advantageous features known in the prior art, including those of the pump described in US—A—4,167,046, and which provides a number of unique and hitherto unknown features which enhance pump reliability and operational characteristics.

A more specific object of the invention is to provide in such a structure a deformable sac having flexing zones which are circumferentially uniform, preferably being entirely defined by machined elements.

A related object of the invention is to provide such a structure in which flexing of the sac during pump operations occurs primarily by a smooth rolling action in the flexing portions of the sac.

Yet another object of the invention is to provide such structure in which creasing and stress-related fatigue in the deformable sac are minimized.

Another object of the invention is to provide such structure designed to produce, during pumping operation a circular flow action which substantially reduces the degree of thrombus formation on inner sac surfaces.

It is still another object of the invention to provide such a chamber structure having a reduced thickness.

The actuator of the invention includes an armature which is mounted for movement between open and closed positions, and which includes a solenoid core defining an internal front-to-back slot. An elongate main spring in the actuator is attached at one spring end to the back region of the armature, extends through the core slot, and is attached at its other end to a pusher element adapted to act against a deformable sac. An elongate preload spring connected at one of its ends to the armature and operatively connected at its other end to the main spring functions to support the main spring in a relatively less stressed condition when the armature is in its open position.

In a preferred embodiment of the invention, the main spring includes a plate-like spring which is curved in the direction of its action on the pusher element, and is in a more planar configuration when in its relatively more stressed condition.

Also in a preferred embodiment of the invention, the actuator includes a symmetrical arrangement of components acting on opposed pusher elements to compress the sac symmetrically from opposite sides. The actuator may further include position sensors for monitoring solenoid armature and/or main spring positions, to provide data used in controlling the operation of the actuator.

The novel chamber structure of the invention comprises, broadly, a deformable sac having an annular side wall which is held in a rigid condition, and a movable wall joined to the side wall through an outwardly convex flexible wall portion. The pusher element, preferably a plate acting against the movable wall, has an initial deflection position at which the flexible wall portion takes the form of a circumferentially uniform, outwardly convex roll having a bulged cross sectional curvature. The roll maintains its circumferential uniformity and bulge-like character as the pusher plate is moved toward inwardly displaced positions to expel fluid from the sac.

In a preferred embodiment of the invention, the annular side wall is held in a rigid condition by attachment to curved inner wall portions of a rigid housing ring. The sac includes a pair of opposed, substantially parallel circular movable walls disposed on opposite sides of the housing ring, each wall being joined to the side wall by an outwardly convex flexible wall portion. A pair of pusher plates acting against associated circular walls are coordinately movable from such initial deflection positions to inwardly displaced positions under the control of an actuator in the pump.

These and other objects and features of the present invention will become more fully apparent when the following detailed description of a preferred embodiment of the invention is read in conjunction with the accompanying drawings.

FIGURE 1 is a side, partially cross-sectional schematic view of a pump employing an actuator constructed in accordance with the invention;

FIGURE 2 is a view like FIGURE 1, with additional parts cut away, illustrating a second condition of the pump;

FIGURE 3 is a view similar to FIGURE 1, with other actuator parts added, illustrating a third condition of the pump;

FIGURE 4 is a top view of the pump shown in FIGURES 1—3;

FIGURE 5 is a sectional view taken generally along line 5—5 in FIGURE 4;

FIGURE 6 is a graph illustrating the force exerted on a pusher plate in the pump by an associated spring beam in the actuator, as a function of the position of the pusher plate between start-of-stroke and end-of-stroke positions;

FIGURE 7 is a schematized plan view, partially cutaway, of a blood pump constructed in accordance with the invention;

FIGURE 8 is a sectional view taken generally along line 8—8 in FIGURE 7;

FIGURE 9 shows in perspective view, a chamber structure in the pump;

FIGURE 10 is a perspective view like that of FIGURE 9, with housing ring and pusher plate parts removed to show the condition of a deformable sac in the structure in a relaxed state (upper part of the figure) and in a start-of-stroke stroke condition (lower part of the figure); and

FIGURE 11 is a view like FIGURE 10, but showing the sac in an end-of-stroke condition in the lower part of the figure.

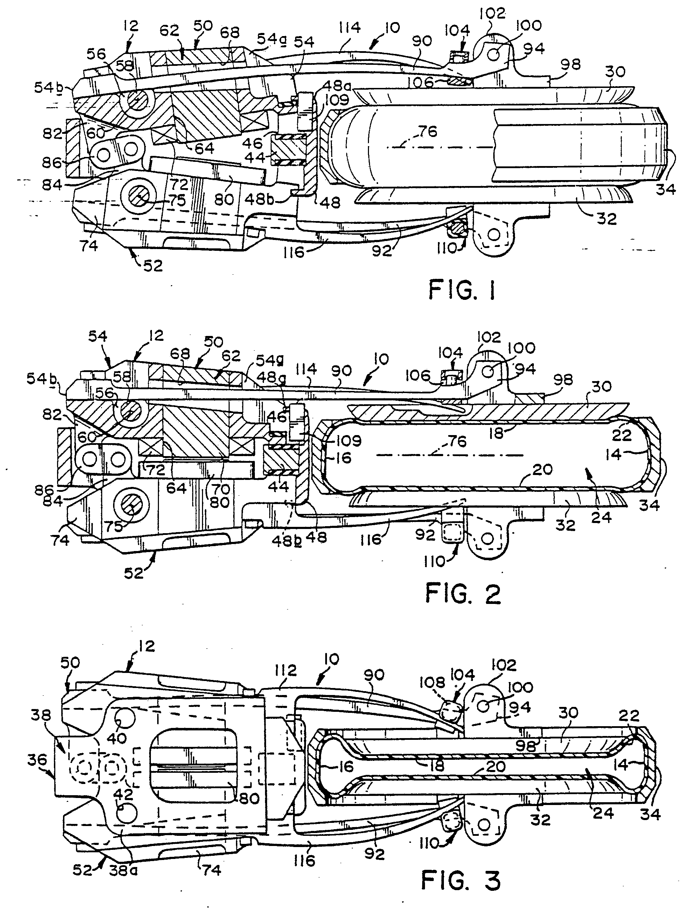

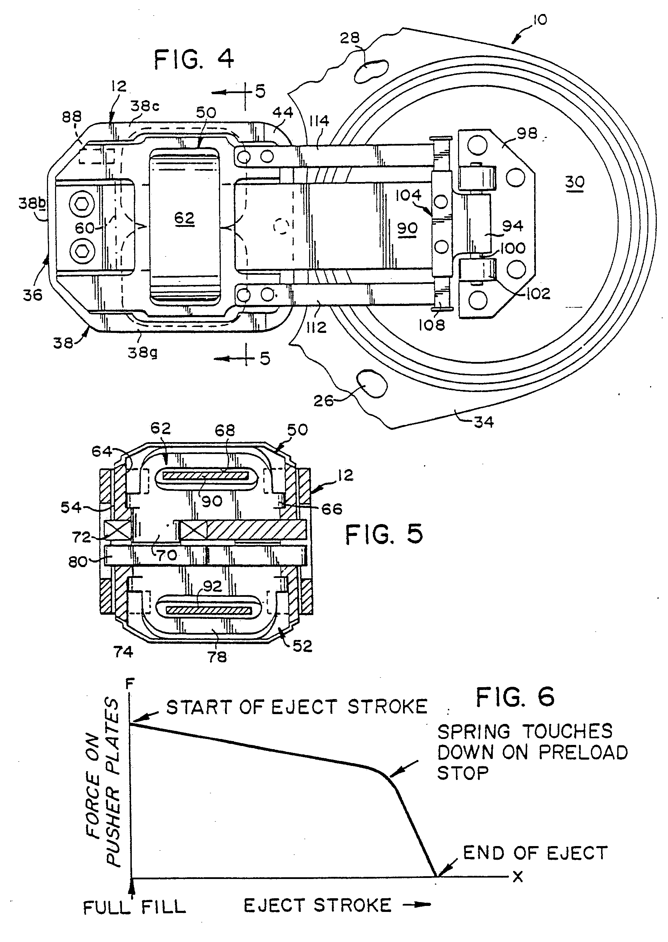

FIGURES 1—4 illustrate an implantable blood pump 10 which is driven by an actuator, or actuator mechanism, 12 constructed according to the present invention. The pump generally includes a deformable sac 14 having an annular side wall 16 (FIGURES 2 and 3) and a pair of opposed circular movable walls 18, 20 each of which is joined to the side wall through an annular flexible wall portion, such as wall portion 22 adjoining wall 18 to wall 16. The sac, which is also referred to as a flexible enclosure, defines an internal variable-volume pump chamber 24. In pump operation, fluid such as blood is supplied to the pump chamber through a valved inlet conduit 26, shown fragmentarily in FIGURE 4, and is expelled under pressure, through a valved outlet conduit, shown fragmentarily at 28 in FIGURE 4.

The pump includes opposed pusher elements, or pusher plates 30, 32 which are attached to, and substantially cover, sac walls 18, 20, respectively. The two pusher plates are movable, under the control of actuator 12, from initial, start-of-stroke positions shown in FIGURE 1 inwardly and synchronously, to end-of-stroke positions shown in FIGURE 3. Such plate movement causes fluid in chamber 24 to be expelled from the pump, through outlet conduit 28.

The central portions of the sac, including side wall 16 and the inlet and outlet conduits formed therewith, are encased in a rigid housing 34. As can be appreciated in FIGURES 2 and 3, the housing functions to support the sac side wall in a stationary position as walls 18, 20 are moved toward and away from one another during pumping action. The rigid housing is also used in attaching actuator 12 rigidly to the pump chamber.

Considering now details of the actuator 12, a frame 36 in the actuator includes a generally U-shaped frame member 38, a side 38a of which is seen in FIGURE 3, and the three sides 38a, 38b, and 38c of which are seen in top view in FIGURE 4. Opposed parallel sides 38a, 38c in the frame member are provided with pairs of aligned bores, such as the pair including bore 40, and the pair including bore 42, both in side 38a, (FIGURE 3).

A front frame plate 44 seen sectionally in FIGURES 1 and 2, and in top view in FIGURE 4 extends between the open sides of the frame member and is secured thereto as by bolting. The upper and lower surfaces of plate 44 are provided with energy absorbing pads, such as pad 46, which serve a purpose to be described. The pads are preferably formed of rubber, polyurethane, or other resilient pad material. Also attached to the frame plate is a stop member 48 having opposed, rearwardly projecting stops 48a, 48b seen in FIGURES 1 and 2.

The actuator includes pair of solenoid armatures 50, 52 mounted on the frame for pivoting between open and closed positions which are shown in FIGURES 1 and 2, respectively. Armature 50, which is representative, comprises an armature support 54 whose front and back end regions are indicated at 54a, 54b, respectively, in FIGURE 1. The armature support is pivotally mounted on frame 36 by a pin 58 which extends through the pair of aligned frame bores which includes bore 40, and is received in bearings, such as bearings 56 held in the armature support. The pivot axis, indicated by dashed-dot line 60 in FIGURE 4, is normal to the direction of movement of the two pusher plates and is also normal to the plane of FIGURES 1—3.

Also included in armature 50 is a C-shaped solenoid core 62 having the general shape seen in side view in FIGURES 1 and 2 and front-on in FIGURE 5. It can be seen in FIGURE 5 that the core is received within a central cavity 64 formed in the support member, with a pair of opposed side projections in the core, such a projection 66, being received against side shoulder portions in the cavity. According to an important feature of the invention, core 62 is provided with an elongate slot 68 extending in a front-to-back direction as seen in FIGURES 1 and 2. The slot serves a purpose which will be described below. Core 62, which is conventionally bonded to the support member, is formed of a suitable ferromagnetic material such as an iron-cobalt-vanadium alloy.

Each of the two inwardly extending poles in the core, such as pole 70, is wrapped with a conductive-wire winding, or coil, such as coil 72 seen cross sectionally in FIGURE 5. The lead wires of the two coils in the core are connected to an actuator control device (not shown) which controls the operation of the actuator in a manner which will be detailed below.

Likewise armature 52 comprises an armature support 74 which is mounted on frame 36 for pivoting about an axis 75 defined by the aligned pair of frame bores which includes bore 42 in FIGURE 3. Axis 75 is parallel to axis 60, and the two axes are equidistant from a mirror image plane which bisects the pump sac axially, and which is indicated by dashed-dot line 76 in FIGURES 1 and 2. Armature 52 also includes a solenoid core 78 having a pair of windings, such as winding 80 seen in FIGURES 1—3, whose lead wires are connected to the above-mentioned control unit. The core windings in the two armatures are energized from a storage capacitor, or by other suitable means in the control unit to set up magnetic fields in the two cores which draw the two armatures together, from their open positions shown in FIGURE 1 toward their closed positions shown in FIGURE 2.

As the armatures approach their closed positions, the armature supports make initial, cushioned contact with associated pads, such as pad 46, on plate 44 as seen in FIGURE 2. These pads cushion solenoid closure at the closed positions of the two armatures, and define a residual solenoid gap between the confronting poles in cores 62, 78. Specifically, the pads are constructed and dimensioned to prevent actual · contact · between the two cores with solenoid closure. The open, unenergized positions of the armatures are determined by the positions of the associated pump pusher plates.

The actuator also includes a mechanical linkage between the two armatures for constraining the armatures to move toward and away from one another symmetrically with respect to the mirror-image plane represented by line 76. This linkage includes a linkage arm 82 formed on the back end of armature support 54, and a more forwardly disposed linkage arm 84 formed on support 74. A connecting link 86 pivotally joins the two arms through pivot pins which are seen cross sectionally in FIGURES 1 and 2. It can be appreciated with reference to the latter figures, that as one of the armatures, for example armature 50, pivots toward its closed positions, movement of arm 82 acts on arm 84 through link 86 to move the other armature substantially the same amount toward its closed position. Similarly, the two armatures move in a symmetrical, coordinated manner when moving from their closed toward their open positions.

The actuator is also provided with a pair of armature position detectors, such as the detector shown schematically in dashed outlines at 88 in FIGURE 4. Preferably each detector is a conventional eddy current type detector which operates to measure the spacing between a current-signaling surface and a target surface, according to current changes produced by magnetic induction in the target surface. In the particular construction herein, each detector is suitably mounted on the rear part of frame 36 to interact with a target surface formed on the associated armature support. The two detectors are operable to provide accurate instantaneous data as to the positions of the associated armatures, as these move between their open and closed positions. The detector position data is supplied to the above-mentioned control device, to provide control feedback information to the control unit during solenoid actuation.

Armatures 50, 52 are operatively connected to associated pusher plates 30, 32 by main springs 90, 92, respectively. Spring 90, which is representative, includes an elongate plate or ribbon-like spring which is seen in side view in FIGURE 1—3 and in plan view in FIGURE 4. The back end of the spring is attached as by bolting to the back end of armature support 54. According to an important feature of the present invention the spring extends through slot 68 formed in armature core 62, as seen in FIGURES 1 and 2. As seen in FIGURE 5, the width of the spring extends along a major portion of the length of the slot. The right end of the main spring in FIGURES 1—4 terminates at an enlargement 94 having a bore formed axially (normal to the long axis of the spring) therein. A pillow block 98 is attached as by bolting to pusher plate 30, as seen best in FIGURE 4. The main spring is pivotally attached to the pillow block by a pivot pin 100 extending through the spring enlargement bore and through bores formed in a pair of mounts, such as mount 102 in the pillow block.

A preload stop member 104 associated with mainspring 90 includes a central slotted portion 106 through which the main spring is received and at which the stop member is secured to the main spring as by bolting. The central portion is seen cross sectionally in FIGURES 1 and 2. Member 104 has a pair of axially opposed preload stops, such as stop 108, each having the general rectangular shape in cross section seen in FIGURE 3.

The main spring is formed of a suitable spring material such as titanium, steel, a fiber composite or the like and is curved, in a relaxed, or unstressed condition in the direction of its action on pusher plate 30, i.e., inwardly on progressing away from the spring's attachment to the armature. As will be seen below with reference to the described operation of the actuator, the curvature of spring 90 seen in FIGURE 1 (and the substantially mirror-image curvature of spring 92) represent the curvature in the spring with such in a relatively less-stressed condition. This curvature is somewhat less than that of the main spring in a totally unstressed or relaxed condition.

The spacing between an inner surface portion of spring 90 and frame 36 is monitored by a position detector 109 similar to the eddy current armature position detectors described above.

The construction of spring 92 and its attachment at one end to armature 52 and at its other end to an associated pusher plate 32 through a pillow block is similar to what has already been described with reference to main spring 90. Also a stop member 110 in the actuator is like stop member 104 in its construction and attachment to the associated main spring.

Completing the description of the actuator, there is associated with each main spring, such as main spring 90, a pair of preload springs, such as springs 112, 114, disposed on either side of the associated main spring as seen in FIGURE 4. The preload springs are attached, as by bolting, to the front end region of support 54 and are dimensioned to contact associated stops, such as stop 108, in the stop member as can be appreciated particularly in FIGURE 3. According to an important feature of the present invention, the preload springs have a selected curvature and spring constant which, with the pump in the condition shown in FIGURE 1, that is, with the solenoid armatures in their open positions, and the pusher plates in their relatively more displaced positions, act to bend spring 90 from its more curved, relaxed position to a less curved condition seen in FIGURES 1 and 3. The action of the preload springs against the main spring, of course, bends the preload springs inwardly somewhat from their relaxed, unstressed positions.

Similar preload springs, such as spring 116 seen in FIGURES 1—3, function to hold main spring 92 in a relatively less stressed position, with the pump in the figuration shown in FIGURE 1, which is a mirror image of the less stressed condition main spring 90. The pair of preload springs associated with each main spring is also referred to herein as preload means. Such means may also include relatively rigid elongate members.

In this condition, each main spring in the actuator is held in its relatively less stressed position by the action of the associated preload springs acting against the stop member on that main spring. Specifically, the preload springs act in an outwardly direction to move the associated main spring from a maximally curved configuration outwardly (away from the mirror image plane in the pump) to the position shown in solid lines in FIGURE 1.

The operation of the pump and particularly the operation of the actuator therein may be observed sequentially in FIGURES 1—3. FIGURE 1 illustrates the pump in a start-of-stroke condition in which pump sac 14 is filled and solenoid armatures 50, 52 are in their open, unenergized positions. The pump ejection stroke is initiated by the control unit, typically with the supply of current to the windings in the armatures from a storage capacitor in the control unit. When energized, the armatures are drawn toward one another, symmetrically with respect to the mirror image plane in the pump, and at a rate which may be controlled by varying the instantaneous current supplied to the windings.

FIGURE 2 shows the condition of the pump immediately after solenoid closure. At this point, the inertia of the filled pump chamber retains the ends of the main springs essentially in the same axially spaced positions as in FIGURE 1. At the same time, the preload springs are moved inwardly with the armatures, out of contact with the stops on the main springs, and the main springs are placed in relatively more stressed, more planar configurations illustrated in solid lines in FIGURE 2. Thus, the energy used in solenoid closure is used initially to produce a greater loading in the main springs, whereby they contain greater stored energy.

After initial solenoid closure, the armatures therein may be held in their closed positions by a relatively small latching current supplied by the control unit. If additional holding force is needed, a small permanent magnet may be used. The force of the latter may be overcome, in returning the armatures to their open positions, by a small reverse current in the solenoid windings.

If the energizing means used to hold the armatures in their closed positions inadvertently fails, with the pusher plates still in their relatively more spaced positions, the relatively greater stress in the main springs may act to open the armatures rapidly beyond their usual open positions. When this occurs, contact between the armatures and associated stops 48a, 48b acts to limit armature "overshoot" to positions only slightly beyond such open positions, and to dampen oscillattory motion in the armatures.

From the condition shown in FIGURE 2, the tendency of the two main springs to relieve the stressed condition therein results in plates 30, 32 being moved toward each other, thus expelling contents of the pump chamber and producing the desired pumping action in the pump. It can be appreciated that as the pusher plates approach their end-of-stroke positions shown in FIGURE 3, the main springs are again positioned to make contact with the associated preload springs through the stops on the main springs. Immediately after contact of the main spring preload stops with the associated preload springs through the stops on the main springs. Immediately after contact of the main spring preload stops with the associated preload springs, a portion of the stored loading energy in the main springs is imparted to the preload springs, bending the latter inwardly until the force and counterforce exerted by the main spring and the preload springs, respectively, are equalized. At this point, the main springs are again in the relatively less stressed, more curved conditions described with respect to FIGURE 1. Once this has occurred, the solenoid coils are deenergized or unlatched.

The apparatus is returned to the condition described with respect to FIGURE 1 as a result of filling of the sac, as by cardiac systole. This filling may take place passively, wherein movement of the pusher plates outwardly to their relatively more spaced positions is accommodated by pivoting of the armatures toward their open positions.

The positions of the pusher plates during the just-described pumping operation may be monitored by position detector 109 which inputs the control unit. Specifically, at the point where the two pusher plates reach their most inwardly moved position, the control unit in the pump may respond to a minimum threshold spacing signal or to a mimimum threshold rate of change signal from the detector to release the latching current in the pump to allow pump filling to occur. During pump filling, as the pump returns from its FIGURE 3 condition to its FIGURE 1 condition, either the detector 105 or the armature position detectors, or both, may signal the control unit, as to a threshold spacing or threshold rate of movement condition which occurs when the pump chamber is filled, and the pump is ready to resume its pumping function.

FIGURE 6 is a graph of the force curve which characterizes the force of a pusher plate, such as plate 30, acting against the associated sac wall during pumping action. The left-hand edge of the curve represents the start of the eject stroke, represented by the FIGURE 2 condition of the pump just after solenoid closure. The nearly horizontal portion of the curve between the start of the eject stroke and the point at which the spring makes contact with the preload stop is the normal characteristic of stress relief in a beam spring between relatively more and relatively less stressed conditions. Each main spring is selected to have a relatively low spring constant so that this portion of the curve is as horizontal as possible. After the main spring makes contact with the preload springs, the force of the pusher plate acting against the sac falls relatively quickly to a zero level as stored energy in the spring is given up to the preload springs and the main spring comes to rest in its relatively less stressed condition.

The construction and operation of the invention has been described above with reference to an actuator having a pair of opposed main springs which are under the control of a pair of coordinately movable armatures. The invention also contemplates an actuator adapted for driving an asymetric deformable pump sac in which the pumping action is achieved by recurrently moving a single pusher plate against a deformable sac surface. Such actuator includes a single armature, like armature 50, connected to the pusher plate operatively by a main spring, such as main spring 90, this being held in a relatively less stressed position during nonpumping phases of the pump operation by one or more preload springs which act on the main spring in the manner described above.

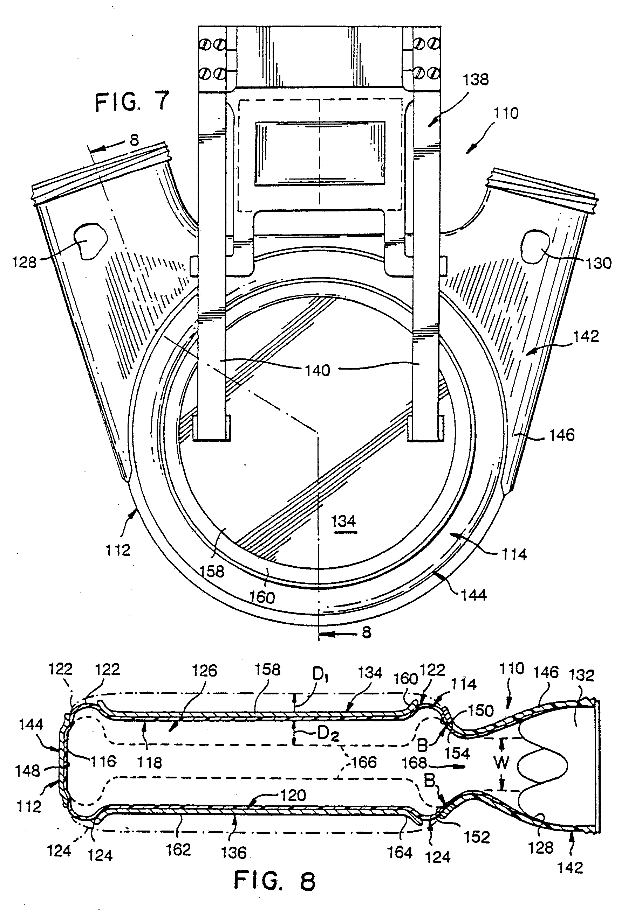

FIGURE 7 shows a view of a pump 110 similar to the pump 10 of FIGURES 1—6. The pump includes the chamber structure which is shown generally at 112 and whose components are shown removed from other parts of the pump. Structure 112, whose construction and unique features will be described in greater detail below, includes the deformable sac 114 having the annular side wall 116, and the pair of opposed circular, movable walls 118, 120 (seen in FIGURE 8) joined to the side wall through flexible curved wall portions 122, 124, respectively. These parts define a variable-volume annular sac chamber, or annulus, 126. Fluid is supplied to the annulus through an inlet conduit 128 and is expelled under pressure, through an outlet conduit 130.

The inlet and outlet conduits are provided with suitable valves, such as inlet valve 132 (FIGURE 8), to produce the requisite one direction flow valving in the pump. Several clinical valves which are commercially available are suitable for use in the pump. In the particular embodiment illustrated herein, 29 mm and 27 mm porcine issue valves are used in the inlet and outlet conduits, respectively.

A pair of opposed pusher plates 134, 136 (FIGURE 8) attached to associated walls 118, 120, respectively, are movable inwardly, under the control of an actuator 138, to produce expulsion of fluid from the sac annulus. The actuator is mechanically connected to each pusher plate through connecting arms, such as arms 140 connecting the actuator to plate 134. Actuator 138, whose design forms no part of the present invention, is preferably an electrically powered solenoid-type actuator which functions to "close" opposed connecting arms coordinately and at a predetermined rate, to produce the desired pumping action in the pump.

Completing the description of what is shown generally in FIGURE 7, pump 110 has a housing 142 which includes a rigid housing ring 144, to be described in detail below, and rigid shell 146 encasing the central region of structure 12, including ring 144. More particularly, the shell encases ring 144, the adjacent side wall region of sac 114, and is formed with passages which accommodate the inlet and outlet conduits in the sac.

The two valves in the pump are held in suitable fittings cast in the housing shell to anchor the valves in place in the deformable sac. The actuator is secured to the housing by suitable means to provide a rigid connection between the actuator and the nondeformable (side wall) portions of the pump sac. The entire pump structure just described may additionally be encased in a fluid tight outer housing (not shown) which is coated with a suitable biocompatible material.

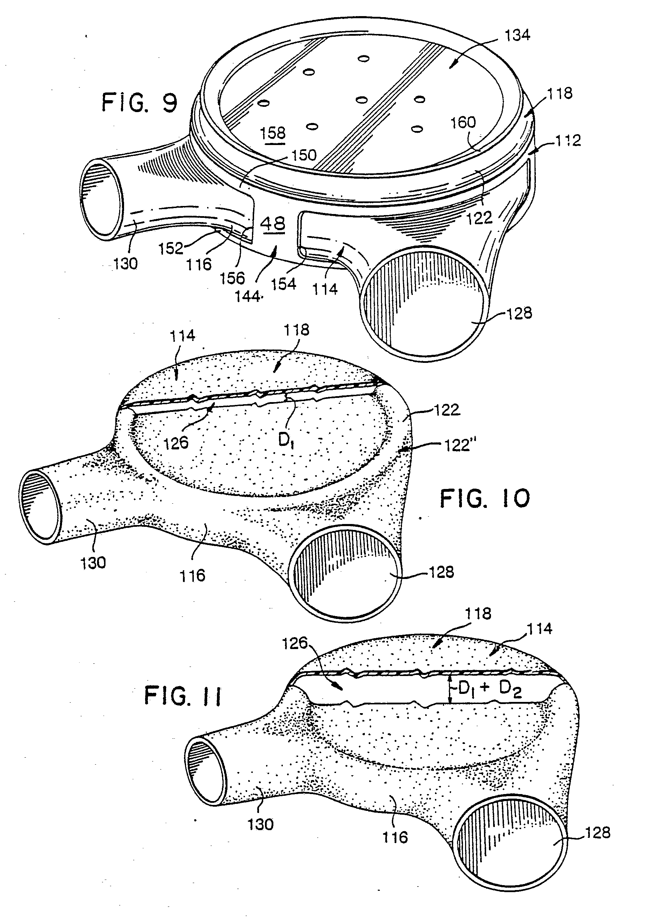

Details of structure 112 will now be considered with particular reference to FIGURES 8—11. Referring first to FIGURES 8 and 9, ring 144 takes the form of an annular band having a substantially straight-walled central portion 148 and a pair of circumferentially continuous annular lip portions 150, 152 having the inwardly curved cross sectional shape seen in FIGURE 8. A pair of slots 154, 156 (FIGURE 9) formed in the ring accommodate inlet and outlet conduits in the sac, respectively. Each slot extends about an approximately 90° arc, with the width of each slot corresponding approximately to the width of the central wall portion in the ring. That is, each slot extends in width substantially between the two opposed annular lip portions in the housing ring. The ring is preferably formed from a lightweight corrosion-resistant material, such as titanium, or a strong rigid polymeric material or the like. The inner surfaces of the curved lip portions in the ring are preferably machined or molded surfaces, for a purpose to be described.

With continued reference to FIGURES 8 and 9, plate 134, which is representative, includes a circular, substantially planar portion 158, and a curved annular lip portion 160 having the cross-sectional curvature seen in FIGURE 8. Plate 136 likewise has a circular planar portion 162 bordered by an annular curved lip portion 164. The pusher plates are preferably formed of a lightweight corrosion-resistant material such as titanium, or of a strong, rigid fiber composite. The inner surfaces of the lip portions in the two pusher plates, like the inner surfaces of the lip portions in the housing ring, are preferably machined or molded, also for a purpose to be described.

While not shown in FIGURES 8 and 9, the pusher plates include mounting structure through which the associated connecting arms such as arms 140, are attached to the associated plates, as can be appreciated with reference to FIGURE 7. According to an important feature of the present invention, the two plates are movable, under the control of actuator 138, acting through the connecting arms, from axially symmetric initial deflection positions, shown in solid lines in FIGURE 8, toward inwardly moved positions. The positions of the inner surfaces of the sac at the end-of-stroke pusher plate positions are shown in dashed lines at 166 in FIGURE 8.

Considering details of sac 14, inlet and outlet conduits 128, 130, respectively, communicate with the sac annulus through elongate ports, such as inlet port 168 (FIGURE 8), formed in the sac. As seen in FIGURE 8, port 168, which is representative, is defined cross-sectionally by a pair of confronting, inwardly convex rolling surfaces which join the annulus and conduit sides of the sac, defining a minimum flow area of width denoted at W in FIGURE 8. Each port, such as port 168, is substantially coextensive, in an arcuate directions, with the corresponding housing ring slot, such as slot 154, through which that part of the sac extends. An advantage of this construction is that a port having a relatively large fluid passage area is formed in an anchored, stationary side wall portion of the sac, whereby the shape of the port is substantially constant during pump operation.

Sac 14 is formed as a unitary seamless article from flexible resilient, blood compatible material. This material may be of any type suitable for pumping blood, such as certain types of polyurethanes. The material of which the sac is comprised should have long-term retention of physical strength under combined dynamic stressing and hydrolysis. The material should be of low toxicity and long-term stability for compatibility with blood. The material should also be of high strength, be capable of being repeatedly flexed, be capable of being sterilized and be easily fabricated. Suitable materials are linear segmented polyurethanes, for example BIOMER from Ethicon.

One preferred method of making the sac comprises successively coating an accurately machined and polished aluminum mandrel whose outer surfaces define the inner surfaces of the sac. Since both of the opposed annular flexible portions in the sac are circumferentially uniform, the surfaces of the mandrel forming such flexible surfaces can be accurately machined, polished, and coated to produce extremely regular and smooth surfaces. To form the sac, the coated mandrel is repeatedly dipped in the selected polymer solution and dried with rotation under infrared lamps. The dipping and drying steps are preferably performed under low humidity conditions. After dipping, the sac is annealed in a vacuum oven. The sac is washed thoroughly in distilled water, solvent extracted and dried in a vacuum oven.

Referring to FIGURE 8, the sac is fitted within ring 144, and flexible sac portions are attached, as with adhesives, to confronting ring lip portions in the region of the ring slots 154, 156. The opposed circular walls in the sac are attached, by a suitable adhesive or the like, to the circular plate portion in the associated pusher plate. The flexing zones of the sac which are not bonded to either the pusher plate or the housing ring are coated with a release agent.

Sac 114 in a relaxed, "as cast" condition, has the general shape seen at the top in FIGURES 10 and 11. In this condition, the circular walls and associated sac flexible portions have the cross-sectional curvature seen in dash-dot lines in FIGURE 8. According to an important feature of the invention, the pusher plates in the pump are placed at symmetrical, initial deflection positions, seen in solid lines in FIGURE 8, at which the circular walls have been moved inwardly toward one another a distance indicated at $D_1$ in the figure. In the particular pump embodiment being described, $D_1$ is preferably about 5.1 mm.

With plates 134, 136 in their initial deflection positions, the associated flexible wall portions in the sac assume the form of circumferentially uniform, outwardly convex rolls, each having the bulged cross-sectional curvature seen in solid lines in FIGURE 8. The rolls formed in wall portions 122, 124 are denoted at 122', 124', respectively in FIGURE 8. It can be appreciated in this figure that the radius of curvature of each roll is substantially less than that of the flexible wall when the sac is in its relaxed condition.

In operation, the two pusher plates are moved, under the control of actuator 138, coordinately and at desired displacement rates, inwardly toward one another from their initial deflection positions toward end-of-stroke positions which place the inner surfaces of the interior of the sac at the positions indicated by the two dashed lines 166 in FIGURE 8. The total working stroke, or distance that each plate is moved, is denoted $D_2$ in FIGURES 8 and 11. In the particular embodiment herein, $D_2$ is about 6.4 mm.

Movement of the pusher plates from their initial deflection positions inwardly is accommodated by a smooth rolling action of the inner and outer annular regions of each roll against the lip portions in the associated pusher plate and housing ring. The sac-to-housing bond lines in the slot regions of the housing ring, indicated at B in FIGURE 8, are located at the point where the sac roll is tangential to the associated housing ring lip portion at the end-of-stroke position. According to an important feature of the invention, and as can be appreciated with reference to FIGURES 8, 10 and 11, each roll in the sac remains circumferentially uniform and bulged in cross-sectional curvature as the pusher plates are moved inwardly from their initial deflection positions. As noted above, the ports communicating the inlet and outlet conduits with the sac annulus are formed entirely within the portion of the sac attached to ring 144. Consequently, the flexing action in the sac occurs substantially independently of, and without effect on, the shape of the ports.

The pusher plates are moved back to their initial deflection position either under the control of the actuator, or passively by inflow of fluid, such as from heart pumping. Sac expansion is accommodated by a smooth, circumferentially uniform rolling action in the sac rolls which characterizes movement of the two rolls during sac contraction.

In flow visualization studies conducted with a pump having clear pusher plates, it was observed that during pump expansion, a circular flow pattern was established which acted to evenly wash the interior surfaces of the sac. In particular, a portion of the flow in the sac is directed back into the inflow conduit to wash the inflow valve. The circular, diastolic flow pattern was very well established and existed until early systole. The improved washing in the valve regions of the pump is due in part to the fact that the inflow conduit is relatively short. The fact that both conduits communicate with the sac annulus through elongate ports whose widths and lengths are substantially fixed during sac contraction and expansion also promotes washing and tangential flow between the conduit regions and the sac annulus.

Analysis of the stress characteristic in the operating pump indicate that the pump design provides a number of improved stress characteristics. Sac stresses were found to be comparable to those in earlier-developed pumps, particularly that described in U.S. Patent No. 4,167,046. However, the stresses in the instant pump are more repeatable and predictable due to the fact that flexing occurs in a region of the deformable sac defined by machined or molded components, this region being itself formed on machined surfaces in the mandrel.

Reduced tendency of the flexing zones to crease is another important feature of the invention. Generally, for a given sac thickness and pusher-plate diameter, the factors which lead to crease formation are long working strokes, and a large housing-to-pusher plate separation. One advantage of the present invention is that due to the predeflection of the pusher plates, the total working stroke in the pump is only about 60% of the total allowed deflection in the sac. Studies on crease formation indicate that, at the pusher plate-housing ring clearance selected, the working stroke in the pump is at least about 10% less than that which can lead to creasing.

From the above, it can be seen how the present invention provides improved operation and reliability in a deformable sac type blood pump. The two annular flexing zones in the pump are each circumferentially uniform and defined by annular machined or molded components to increase stability and circular flow characteristics. The predeflection feature in the pump functions to create a bulged annular roll in each of the flexing zones, which can accommodate recurrent sac wall movement by a smooth rolling action that minimizes localized stressed and the tendency to crease over extended operation.

The predeflection feature in the pump produces two other significant advantages. First, the thickness of the pump chamber structure, with such in a filled, start-of-stroke condition, is considerably reduced over the thickness the structure would have with the sac in a fully expanded condition. The reduced thickness is two time $D_1$, or about 10 mm in the particular pump described herein. The advantage of the reduced pump thickness in an implantable pump can be appreciated. Secondly, a relatively small internal volume in the pump chamber, at the end of stroke, can be achieved with a relatively small total stroke. The small internal volume promotes "flushing" of fluid from the pump, thereby reducing the potential for thrombus formation.

The problem of thrombus formation in the sac is reduced both by the improved flow characteristics which are observed during pump operation, and by the features of the sac construction which promote washing in the conduit regions of the sac. In this regard, the elongate ports communicating the conduits with the annulus in the sac and the relatively short inflow valve are important.

While a preferred embodiment of the invention has been described herein, it will be evident to those skilled in the art that various changes and modifications may be made without departing from the spirit of the invention. In particular, the invention also contemplates an asymetric chamber structure in which pumping occurs through movement of a single wall, where such movement is accommodated by the rolling action of a circumferentially uniform bulged roll, as described.

The invention also contemplates a sac which is formed to have, in a relaxed, or unstressed, condition, the general cross-sectional shape seen in solid lines in FIGURE 8. That is, the sac, as cast, is formed to have a pair of opposed recessed circular walls bordered by a circumferentially uniform roll having a bulged cross-sectional curvature. The sac may also be constructed to have an annular side wall which is, by its construction, inherently rigid and therefore does not require external rigidifying structure, such as the housing ring described.

From the foregoing, it can also be seen how the advantageous features of the pump actuator mechanism described in the above-mentioned U.S. patents are improved upon. These advantages include mechanical simplicity, and greater efficiency and reliability inherent in the operation of a beam spring acting between relatively less stressed and relatively more stressed conditions and directly coupled to a solenoid armature and a pusher plate acting on a deformable sac.

The present invention provides additional advantages which are not present in pump actuators disclosed in the prior art. One important advantage is that the unique armature construction, and in particular, the slotted armature core in the actuator, allows the armature to be operatively coupled to the associated pusher plate by a single, relatively wide main spring extending from the back of the armature through the solenoid core slot to its point of attachment on the pusher plate. A significant advantage in the use of the single main spring is that problems of matching the spring constant characteristics of two or more springs working in parallel are avoided, as are problems of nonuniform changes in spring performance characteristics over extended periods of pump use.

The actuator construction also allows for greater compactness. In this regard, it is noted that the placement of a portion of the main spring within the solenoid core in the armature permits the main spring to be disposed relatively close to the mirror image plane in the actuator. Further, the inward curvature of the main spring and the preload springs in the actuator act to reduce overall pump thickness.

Symmetry of pump design and operation largely eliminates loads between the actuator and the pump housing. The linkage mechanism insures that movement of the armatures is symmetrical, particularly during pump filling. Because the armatures operate in a symmetrical manner, information about the position of one armature can be used to provide accurate position information as to both armatures.

**Claims**

1. In a pump having a flexible enclosure defining a pump chamber, and a pair of opposed substantially axially aligned pusher elements engaged with opposed movable walls in the enclosure for movement between first relatively more spaced positions and second relatively less spaced positions to expel fluid from the enclosure, an actuator for producing coordinated movement of the pusher elements between their first and second positions, comprising a frame, a pair of armatures mounted on said frame for movement between open relatively more spaced and closed relatively less spaced positions, a solenoid core in each armature, said solenoid cores being energizable to cause said armatures to move from their open positions to their closed positions, a pair of opposed elongate main springs each attached at one spring end to an associated armature and being attached at its other spring end to an associated pusher element, said main springs being arranged on opposite sides of the flexible enclosure and being arranged within the outermost dimension of said armatures, and elongated preload means associated with each of said main springs, each of said preload means having one end connected to the associated armature and having its other end arranged for operative engagement with the associated main spring for holding said main spring in a relatively less stressed condition with the pusher element in its first position and with said armature in its open position, said armatures being movable to their closed positions with the pusher elements still in their first positions to disengage said preload means from said associated main springs and bend the main springs to relatively more stressed second conditions, whereby in relieving the increased stress in said main springs, the ends of said main springs are displaced to move the pusher elements coordinately toward their second relatively less spaced positions, said actuator being characterized in that said solenoid cores each have a front-to-back slot therein with a respective one of said main springs extending through said slot, said slots each being of a size sufficient to provide clearance between each of said cores and the associated one of said main springs for all said operative positions of said armatures and all said operative conditions of said springs.

2. The actuator of Claim 1, wherein each main spring comprises a plate-like spring which, in its relatively less stressed condition, is curved in the direction of its action on the associated element, and in its relatively more stressed conditions, is relatively more planar.

3. The actuator of Claim 1, wherein each of said preload means includes a pair of springs disposed on either side of the associated main spring.

4. The actuator of Claim 1, which further includes position detector means for measuring the spacing between said armatures throughout movement between their open and closed positions.

5. The actuator of Claim 4, wherein said position detector means includes means for determining the spacing between said frame and a reference point on one of said armatures.

6. The actuator of Claim 1, which further includes position detector means for detecting the position of at least one pusher element by measuring the spacing between the associated main spring and said frame throughout relative movement therebetween.

7. The actuator of Claim 1, wherein said armatures are mounted on said frame for pivoting about spaced parallel axes, and are pivotly connected to one another to produce substantially symmetrical, coordinated movement of the armatures with respect to a plane disposed midway between, and normal to the plane containing said axes.

**Patentansprüche**

1. In einer Pumpe mit einer flexiblen umhüllung, die eine Pumpenkammer bildet, und einem Paar entgegengesetzter und im wesentlichen axial ausgerichteter Stoßelementen, die in jeweils gegenüberliegende bewegliche Wände der Umhüllung eingreifen und diese zwischen verhältnismäßig weit auseinanderliegenden Stellungen einerseits und zwischen Stellungen mit verhältnismäßig wenig Abstand andererseits in Bewegung halten, um eine Flüssigkeit aus der Umhüllung herauszutreiben, sowie einem Stellantrieb zur Erzeugung einer aufeinander abgestimmten Bewegung der Stoßelemente zwischen den einen und den anderen Stellungen, bestehend aus einem Rahmen, einem Paar auf dem erwähnten Rahmen installierter Läufer zur Bewegung zwischen den im geöffneten Zustand verhältnismäßig weit auseinanderliegenden und den im geschlossenen Zustand mit verhältnismäßig wenig Abstand anliegenden Stellungen, einem Tauchmagnetkern in jedem Läufer, wobei die erwähnten Tauchmagnetkerne elektrisch erregt werden können, um die erwähnten Läufer sich aus ihren geöffneten Stellungen in ihre geschlossenen Stellungen bewegen zu lassen, einem Paar entgegengesetzter Dehnzugfedern, von denen das eine Ende der jeweiligen Feder an einem dazugehörigen Läufer und das andere Ende der jeweiligen Feder an einem dazugehörigen Stoßelement befestigt ist, wobei die erwähnten Zugfedern an den jeweils gegenüberliegenden Seiten der flexiblen Umhüllung sowie innerhalb der äußersten Ausdehnung der erwähnten Läufer angeordnet sind, und zu jeder der erwähnten Zugfedern zugehörige Dehnungsvorspannvorrichtungen, wobei jede der erwähnten Vorspannvorrichtungen mit einem Ende mit dem dazugehörigen Läufer verbunden ist und ihr anderes Ende derart angerodnet ist, daß es bei dem Betrieb in die dazugehörige Zugfeder eingreift, um die erwähnte Zugfeder in einem verhältnismäßig gering belasteten Zustand zu halten, wenn sich das Stoßelement in der ersten Stellung und der erwähnten Läufer in seiner geöffneten Stellung befindet, wobei die erwähnten Läufer sich in ihre geschlossene Stellung bewegen können, während die Stoßelemente noch in ihrer jeweiligen ersten Stellung verbleiben, um die erwähnten Vorspannvorrichtungen aus den dazugehörigen Zugfedern zu lösen und die Zugfedern jeweils in einen verhältnismäßig stärker spannungsbelasteten zweiten Zustand zu biegen, wobei durch den Abbau der erhöhten Spannung in den erwähnten Zugfedern sich die Enden der erwähnten Zugfedern verschieben, um die Stoßelemente aufeinander abgestimmt zu ihrer jeweiligen zweiten Stellung mit verhältnismäßig wenig Abstand zu bewegen, wobei der erwähnte Stellantrieb dadurch gekennzeichnet ist, daß in jedem der erwähnten Tauchmagnetkerne sich ein von vorne bis hinten reichender Schlitz befindet, durch den sich jeweils eine der erwähnten Zugfedern erstreckt, wobei jeder der erwähnten Schlitze von ausreichender Größe ist, daß bei jedem der erwähnten Betriebszustände der erwähnten Läufer sowie bei jedem der erwähnten Betriebszustände der erwähnten Federn zwischen jedem der erwähnten Kerne und der erwähnten jeweils dazugehörigen Zugfeder ein ausreichender Spielraum bereitsteht.

2. Stellantrieb nach dem Patentanspruch 1, wobei jede Zugfeder eine tellerähnliche Feder enthält, die in ihrem verhältnismäßig gering belasteten Zustand in die Richtung ihrer Wirkung auf ein dazugehöriges Element gebogen wird und in ihrem verhältnismäßig stärker spannungsbelasteten Zustand verhältnismäßig ebener ist.

3. Stellantrieb nach dem Patentanspruch 1, wobei zu jeder der erwähnten Vorspannvorrichtungen ein Paar Federn gehört, die zu jeder Seite der dazugehörigen Zugfeder angerodnet sind.

4. Stellantrieb nach dem Patentanspruch 1, zu dem ferner eine Stellungsgebervorrichtung gehört, um den Abstand zwischen den erwähnten Läufern im Verlauf der Bewegung zwischen ihrer jeweiligen geöffneten und geschlossenen Stellung zu messen.

5. Stellantrieb nach dem Patentanspruch 4, wobei zu der erwähnten Stellungsgebervorrichtung eine Vorrichtung zur Bestimmung des Abstands zwischen dem erwähnten Rahmen und einem Bezugspunkt auf einem der erwähnten Läufer gehört.

6. Stellantrieb nach dem Patentanspruch 1, zu dem ferner eine Stellungsgebervorrichtung gehört, um die Stellung von mindestens einem Stoßelement durch Messen des Abstands zwischen der dazugehörigen Zugfeder und dem erwähnten Rahmen im Verlauf ihrer jeweils zueinander ablaufenden Bewegung zu ermitteln.

7. Stellantrieb nach dem Patentanspruch 1, wobei die erwähnten Läufer auf dem erwähnten Rahmen installiert sind, um sich um parallele Achsen mit räumlichem Abstand zueinander schwenken zu lassen, und schwenkbar miteinander verbunden sind, um eine in Bezug auf eine in ihrer Mitte angeordnete Ebene im wesentlichen symmetrische, aufeinander abgestimmte und zu der aus diesen Achsen gebildeten Ebene lotrechte Bewegung der Läufer zu erzeugen.

**Revendications**

1. Dans une pompe dotée d'une enceinte flexible délimitant une chambre de pompe, et une paire d'éléments pousseurs opposés essentiellement alignés l'un sur l'autre, avec des parois mobiles opposées dans l'enceinte pour le mouvement entre des premières positions relativement plus espacées et des deuxièmes positions relativement moins espacées pour expulser le fluide de l'enceinte, un dispositif de commande est prévu pour produire le mouvement coordonné des éléments pousseurs entre leurs premières et deuxièmes positions, comprenant un bâti, une paire d'induits montés sur ledit bâti pour déplacement entre les positions ouvertes relativement plus espacées et les positions fermées relativement moins espacées, un noyau de solénoïde dans chaque induit, lesdits noyaux de solénoïde étant excitables pour effectuer le déplacement desdits induits de leurs positions ouvertes vers leurs positions fermées, une paire de ressorts principaux allongés opposés, chacun d'eux étant relié, à une extrémité, à un induit associé et étant relié, à son autre extrémité, à un élément pousseur associé, lesdits ressorts principaux étant disposés sur des côtés opposés de l'enceinte flexible et étant placés endéans de la dimension extrême desdits induits, et des moyens de préchargement allongés associés à chacun desdits ressorts principaux, chacun desdits moyens de préchargement ayant une extrémité reliée à l'induit associé et ayant son autre extrémité conçue pour se mettre activement en prise avec le ressort principal associé pour tenir ledit ressort principal dans un état relativement moins sollicité, l'élément pousseur se trouvant en sa première position et ledit induit se trouvant en sa position ouverte, lesdits induits pouvant être déplacés vers leurs positions fermées avec les éléments pousseurs se trouvant toujours en leurs premières positions pour désenclencher lesdits moyens de préchargement desdits ressorts principaux associés et fléchir les ressorts principaux vers des deuxièmes états relativement plus sollicités, à la suite de quoi, par le relâchement de la sollicitation augmentée dans lesdits ressorts principaux, les extrémités desdits ressorts principaux sont déplacées pour effectuer le mouvement coordonné des éléments pousseurs vers leurs deuxièmes positions relativement moins espacées, ledit dispositif de commande étant caractérisé en ce que lesdits noyaux de solénoïdes ont chacun intérieurement une rainure d'avant en arrière avec un desdits ressorts principaux s'étendant respectivement au travers de ladite rainure, lesdites rainures ayant chacune des dimensions suffisantes pour assurer un espacement entre chacun desdits noyaux et le ressort associé desdits ressort principaux pour toutes lesdites positions actives desdits induits et tous lesdits états actifs desdits ressorts.

2. Le dispositif de commande selon la revendication 1, caractérisé en ce que chaque ressort principal comprend un ressort en forme de lame qui, en son état relativement moins sollicité, est recourbé dans le sens de son action sur l'élément associé et qui, dans ses états relativement moins sollicités, est relativement plus plan.

3. Le dispositif de commande selon la revendication 1, caractérisé en ce que chacun desdits moyens de préchargement comprend une paire de ressorts disposés de chaque côté du ressort principal associé.

4. Le dispositif de commande selon la revendication 1, caractérisé en ce qu'il comprend en outre des moyens détecteurs de position pour mesurer l'espacement entre lesdits induits dans tout le déplacement entre leurs positions ouvertes et fermées.

5. Le dispositif de commande selon la revendication 4, caractérisé en ce que lesdits moyens détecteurs de position comprennent des moyens de détermination de l'espace entre ledit bâti et un point de repère sur l'un desdits induits.

6. Le dispositif de commande selon la revendication 1, caractérisé en ce qu'il comprend en outre des moyens détecteurs de position pour détecter la position d'au moins un élément pousseur par la mesure de l'espacement entre le ressort principal associé et ledit bâti dans tout le déplacement relatif entre ressort et bâti.

7. Le dispositif de commande selon la revendication 1, caractérisé en ce que lesdits induits sont montés sur ledit bâti pour pivotement autour d'axes parallèles espacés et en ce qu'ils sont reliés ensemble à pivotement pour produire un déplacement coordonné essentiellement symétrique des induits par rapport à un plan disposé à mi-chemin entre eux, et perpendiculairement au plan contenant lesdits axes.

FIG. 1

FIG. 2

FIG. 3

FIG. 4

FIG. 5

FIG. 6

START OF EJECT STROKE

SPRING TOUCHES
DOWN ON PRELOAD
STOP

FORCE ON PUSHER PLATES

END OF EJECT

FULL FILL          EJECT STROKE →

FIG. 7

FIG. 8

FIG. 9

FIG. 10

FIG. 11

4